# EUROPÄISCHE PATENTSCHRIFT

(11) **EP 4 151 324 B1**
(45) Veröffentlichungstag und Bekanntmachung des Hinweises auf die Patenterteilung: **29.05.2024**
(21) Anmeldenummer: 22194768.2
(22) Anmeldetag: 09.09.2022
(51) Int. Cl.: B08B 9/027, B08B 11/02, A61M 39/08, A61C 19/00

(54) **AUFNAHMEVORRICHTUNG ZUM AUFNEHMEN MINDESTENS EINES SCHLAUCHS FÜR EIN REINIGUNGSGERÄT UND REINIGUNGSGERÄT**
RECEIVING DEVICE FOR RECEIVING AT LEAST ONE HOSE FOR A CLEANING DEVICE AND CLEANING DEVICE
DISPOSITIF DE RÉCEPTION POUR RECEVOIR AU MOINS UN TUYAU FLEXIBLE POUR UN APPAREIL DE NETTOYAGE ET APPAREIL DE NETTOYAGE

(30) Priorität: 16.09.2021 DE 102021123943
(43) Veröffentlichungstag der Anmeldung: 22.03.2023
(73) Patentinhaber: Miele & Cie. KG, 33332 Gütersloh (DE)
(72) Erfinder: Geddes, Marita, 33619 Bielefeld (DE); Weber, Ulrike, 33609 Bielefeld (DE); Restemeier, Dennis, 33332 Gütersloh (DE); Neufeld, Roman, 33619 Bielefeld (DE); Crilley, Ivana, 33335 Gütersloh (DE); Voßhans, Ralf, 33335 Gütersloh (DE)

(56) Entgegenhaltungen:
- EP-A1- 0 679 406
- EP-A1- 2 008 608
- WO-A1-97/39609
- WO-A1-2006/062457
- DE-A1- 3 443 912
- DE-A1-102012 100 881
- DE-A1-102014 205 547
- DE-A1-102018 102 243
- DE-C2- 3 710 349
- US-A- 543 215
- US-A- 4 980 536
- US-A- 5 669 970
- US-A1- 2014 248 443
- US-B1- 6 382 575

## Beschreibung

Die Erfindung betrifft eine Aufnahmevorrichtung für ein Reinigungsgerät, welche zum Aufnehmen mindestens eines Schlauchs dient, und ein Reinigungsgerät.

Reinigungsgeräte sind beispielsweise in einer Vielzahl von Bereichen einsetzbar, beispielsweise in Form von Spülmaschinen, Waschmaschinen oder in Form von Staubsaugern, die beispielsweise gewerblich oder privat einsetzbar sind. Reinigungsgeräte sind demnach auch im medizinischen Bereich verwendbar. Für die maschinelle Reinigung und Desinfektion von medizinischen Schläuchen für beispielsweise Lachgasanwendungen sollten diese im Beladungsträger eines Reinigungs- und Desinfektionsgeräts (RDG) adaptiert, gehalten und positioniert werden. Dabei ist es für die Reinigung wichtig, dass die Schläuche steigend verlegt werden, damit sich keine Spülflotte ansammelt und die Spülflotte frei auslaufen kann.

Die EP 0 679 406 A1 offenbart eine Aufnahmeeinrichtung für ein Reinigungsgerät, welche zur Aufnahme eines innen zu durchspülenden zahnärztlichen Instruments dient. Diese weist einen Bottich auf, an dem ein Anschluss für ein zu spülendes Instrument angeordnet ist.

Die DE 10 2018 102 243 A1 offenbart eine Aufnahmevorrichtung für ein Reinigungsgerät zum Einsetzen in ein Korbelement des Reinigungsgeräts, wobei die Aufnahmevorrichtung mehrere Halteelemente umfasst, die jeweils eine Mehrzahl von Durchgangsöffnungen aufweisen sowie mehrere, jeweils in eines der Halteelemente einsetzbare Aufnahmeeinrichtungen, die jeweils zur Aufnahme eines Abschnitts eines Endoskops dienen.

Die US 6,382,575 B1 offenbart ein Einsetzelement, das zur Halterung von medizinischen Instrumente dient, und an ein Korbelement angeklipst werden kann.

Der hier vorgestellte Ansatz stellt sich die Aufgabe, eine verbesserte zum Aufnehmen mindestens eines Schlauchs dienende Aufnahmevorrichtung für ein Reinigungsgerät sowie ein verbessertes Reinigungsgerät zu schaffen.

Erfindungsgemäß wird diese Aufgabe durch eine Aufnahmevorrichtung für ein Reinigungsgerät, die zum Aufnehmen mindestens eines Schlauchs dient, sowie durch ein Reinigungsgerät mit den Merkmalen der Hauptansprüche gelöst. Vorteilhafte Ausgestaltungen und Weiterbildungen der Erfindung ergeben sich aus den nachfolgenden Unteransprüchen.

Die durch den Ansatz erreichbaren Vorteile bestehen in einer modularen Nutzung des vorgestellten Ansatzes, der nach Belieben eines Nutzers genutzt werden kann. Weiterhin kann innerhalb des Reinigungsgeräts beispielsweise Beladungsfläche effektiv genutzt werden. Der Ansatz beschreibt eine flexible, ergonomische und ökonomische Lösung für eine verbesserte Aufbereitung für Schläuche. Eine benötigte Beladungsfläche für eine Aufbereitung der Schläuche kann beispielsweise reduziert werden.

Es wird eine Aufnahmevorrichtung gemäß Anspruch 1 vorgestellt.

Die Aufnahmevorrichtung ist für ein Reinigungsgerät realisiert, das beispielsweise zum Reinigen von medizinischen Werkzeugen eingesetzt werden kann. Die Aufnahmevorrichtung ist zum Einsetzen in ein Korbelement des Reinigungsgeräts und zum Aufnehmen eines Schlauchs eingerichtet.

Der mindestens eine Schlauch kann beispielsweise auch als Schlauchset realisiert sein, das eine Mehrzahl von (Teil-) Schläuchen aufweisen kann, wie sie beispielsweise im Bereich der Zahnmedizin Anwendung finden, beispielsweise in Verbindung mit einer Lachgassedierung. Die Aufnahmevorrichtung kann vorteilhafterweise je nach Bedarf und zusätzlich oder alternativ einer Menge des Reinigungsguts durch einen Anwender in ein vorhandenes Reinigungsgerät eingesetzt werden oder entnommen werden. Die Gerüsteinheit beinhaltet eine Mehrzahl von Gerüstelementen, die vorteilhafterweise stangenartig ausgeformt sein können, und eine Mehrzahl von Halteelementen, die beispielsweise jeweils mindestens eine Öffnung aufweisen können. Die Gerüstelemente können dabei beispielsweise durch diese Öffnungen der Halteelemente gesteckt sein oder werden und somit vorteilhafterweise ein Gerüst bilden, welches auch wieder leicht demontiert und somit bei einer Nicht-Benutzung platzsparend gelagert werden kann. Das Einsetzelement kann beispielsweise in das Halteelement eingehakt, eingeklipst und zusätzlich oder alternativ eingeschoben werden. Der Aufnahmebereich kann dabei als ein Ausschnitt des Einsetzelements ausgeformt sein. Vorteilhafterweise kann pro Halteelement ein Einsetzelement eingesetzt werden. Bei einer Mehrzahl von Halteelementen sind dementsprechend eine Mehrzahl von Einsetzelementen als Teil der Aufnahmevorrichtung ausgeformt. Die einzelnen Einsetzelemente können dabei vorteilhafterweise auf unterschiedlichen Höhenpositionen an den Halteelementen oder bezüglich der Halteelemente angeordnet sein, sodass der Schlauch vorteilhafterweise abschüssig in der Aufnahmevorrichtung angeordnet werden kann. Vorteilhafterweise kann dadurch beispielsweise eine in dem Schlauch befindliche Reinigungsflotte nach einem Reinigungsvorgang abfließen. Das Verbindungselement kann beispielsweise als ein Adapter fungieren, das mit der Spülleiste verbunden, beziehungsweise angeschlossen ist oder werden kann und über das die Reinigungsflotte in den Schlauch zum Reinigen des Schlauchs eingeleitet werden kann. Die Spülleiste kann dabei beispielsweise in dem Korbelement des Reinigungsgeräts fest angeordnet sein. Vorteilhafterweise kann die Aufnahmevorrichtung mindestens ein weiteres Verbindungselement aufweisen, das ausgeformt ist, um einen weiteren Schlauch mit der Spülleiste zu verbinden. Vorteilhafterweise können pro Schlauchset bzw. Schlauch zwei Verbindungselemente in dem Reinigungsgerät angeordnet sein. Vorteilhafterweise kann der mindestens eine Schlauch durch die modular gestaltete Aufnahmevorrichtung sicher und steigend positioniert werden, sodass beispielsweise überschüssige Reinigungsflüssigkeit leichter abfließen kann. Das Verbindungselement kann vorteilhafterweise universell für eine Vielzahl unterschiedlich ausgeformter Schläuche mit unterschiedlichen Anschlüssen verwendet werden, beispielsweise durch den Einsatz einer Formdichtung.

Gemäß einer Ausführungsform kann das Einsetzelement an den Aufnahmebereich angrenzend eine Stütznase aufweisen, insbesondere wobei die Stütznase einteilig mit dem Einsetzelement ausgeformt ist. Zusätzlich oder alternativ kann die Stütznase durch einen umgebogenen Stanzausschnitt des Einsetzelements ausgeformt sein. Die Stütznase kann vorteilhafterweise ausgebildet sein, um den in die Aufnahmevorrichtung eingelegten Schlauch zu stützen und somit beispielsweise eine abschüssige Lagerung zu realisieren. Auch können hierdurch beispielsweise Druckstellen vermieden werden, die bei einem Reinigungsvorgang beispielsweise zu einer Störung eines Flusses der Reinigungsflotte führen können. Die Stütznase kann beispielsweise abgerundete Ecken aufweisen, sodass vorteilhafterweise eine Verletzung des Schlauchs oder von weiterem Reinigungsgut vermieden werden kann.

Das Einsetzelement weist in einem ersten Randbereich ein erstes Einhakelement und in einem zweiten Randbereich ein zweites Einhakelement aufn, die ausgebildet sind, um das Einsetzelement in das Halteelement einzuhaken. Die Einhakelemente können dabei beispielsweise quer zu einer Haupterstreckungsachse des Einsetzelements angeordnet sein. Weiterhin können die Einhakelemente eckig ausgeformt sein.

Ferner weist jedes der Halteelemente eine Mehrzahl von Durchgangsöffnungen auf, die in einer Reihe und auf gegenüberliegenden Schenkeln des Halteelements angeordnet sind. Die Durchgangsöffnungen können beispielsweise als Stanzlöcher oder als Bohrlöcher ausgeformt sein, die vorteilhafterweise übereinander angeordnet sein können. Die Durchgangsöffnungen können beispielsweise durch Bohren oder durch Stanzen in das Halteelement gebracht worden sein, sodass die Durchgangsöffnungen beispielsweise als runde, abgerundete oder beispielsweise als eckige Öffnungen realisiert sind. Vorteilhafterweise kann das Einsetzelement in die Durchgangsöffnungen eingehakt und somit auf einer Position gehalten werden. Die Position ist dabei flexibel durch den Nutzer des Reinigungsgeräts veränderbar. Durch die Verwendung einer Mehrzahl von Durchgangsöffnungen können auch die Einsetzelemente an den einzelnen Halteelemente in unterschiedlicher Höhe eingehakt werden, sodass nur ein standardisiertes Halteelement hergestellt werden braucht, welches für den Einsatz an unterschiedlichen Positionen in der Aufnahmevorrichtung konfiguriert werden kann.

Gemäß einer Ausführungsform kann das Einsetzelement mindestens einen weiteren Aufnahmebereich aufweisen, der ausgebildet ist, um einen weiteren Schlauch zu halten. Der weitere Aufnahmebereich kann beispielsweise auf einer Ebene mit dem Aufnahmebereich liegen oder beispielsweise auf einer parallelen Ebene zu dem Aufnahmebereich. Die Aufnahmebereiche können dabei beispielsweise gleichartig ausgeformt sein. Vorteilhafterweise kann dadurch eine Mehrzahl von Schläuchen und/oder von Schlauchsets aufgenommen werden, die zeitgleich gereinigt werden können. Vorteilhafterweise kann ein Reinigungsvorgang dadurch effizient und wassersparend gestaltet werden, da eine größere Menge von Reinigungsgut gleichzeitig gereinigt werden kann.

Weiterhin kann das Einsetzelement zwischen dem Aufnahmebereich und dem mindestens einen weiteren Aufnahmebereich einen Trennsteg zum Trennen der Aufnahmebereiche aufweisen. Der Trennsteg kann beispielsweise in Richtung einer Öffnung der Aufnahmebereiche zeigen. Beispielsweise kann der Trennsteg pyramidenartig oder alternativ pilzartig ausgeformt sein. Bei einer pilzartigen Form des Trennstegs kann der Schlauch oder das Schlauchset vorteilhafterweise in einer gewünschten Position gehalten werden, da der Trennsteg in Richtung eines freien Endes aufgeweitet ausgeformt und somit eine Haltefunktion übernehmen kann.

Gemäß einer Ausführungsform kann der Trennsteg Y-förmig ausgeformt sein. Vorteilhafterweise kann der Y-förmige Trennsteg einen der weiteren Aufnahmebereiche ausformen, der auf einer parallelen Ebene zu dem mindestens einen Aufnahmebereich angeordnet sein kann. In Richtung des freien Endes des Trennstegs kann der Trennsteg dabei zangenartig ausgeformt sein, um einen weiteren Schlauch aufnehmen zu können.

Die Aufnahmevorrichtung kann ferner ein Erweiterungselement aufweisen, das in das Einsetzelement einsetzbar sein kann, wobei das Erweiterungselement mindestens einen Erweiterungsaufnahmebereich aufweisen kann, der ausgebildet ist, um mindestens einen zusätzlichen Schlauch zu halten. Das Erweiterungselement kann beispielsweise in das Einsetzelement eingeschoben werden und kann den Erweiterungsaufnahmebereich zum Aufnehmen des zusätzlichen Schlauchs aufweisen. Beispielsweise kann das Erweiterungselement den oder einen weiteren Y-förmigen Trennsteg aufweisen, sodass vorteilhafterweise in zusammengesetztem Zustand des Einsetzelements und des Erweiterungselements bis zu fünf Schläuche oder Schlauchsets aufgenommen werden können. Vorteilhafterweise kann dadurch ein Wasserverbrauch für einen Reinigungsvorgang reduziert werden, da eine größere Anzahl von Schläuchen zeitgleich gereinigt werden können.

Gemäß einer Ausführungsform kann das Einsetzelement mindestens ein Einklipselement zum Befestigen des Erweiterungselements an dem Einsetzelement aufweisen. Vorteilhafterweise kann das Einsetzelement mindestens zwei Einklipselemente aufweisen, die an einander gegenüberliegenden Seiten des Einsetzelements angeordnet sein können. Die Einklipselemente können beispielsweise C-förmig realisiert sein. Dabei können die Einklipselemente beispielsweise in zwei sich voneinander unterscheidende Richtungen geöffnet sein, insbesondere kann die Öffnung eines der Einklipselemente in Richtung des anderen Einklipselements zeigen. Vorteilhafterweise kann das Erweiterungselement durch eine Schwenkbewegung mit dem Einsetzelement verbunden werden. Dabei kann beispielsweise ein Klipsvorsprung des Erweiterungselements in eines der Einklipselemente eingeklipst werden, worauf die Schwenkbewegung folgen kann, um einen zweiten Klipsvorsprung in das andere Einklipselement einzuklipsen.

Weiterhin kann das Verbindungselement mit der Spülleiste verbunden, insbesondere verschraubt sein. Vorteilhafterweise kann durch ein Verschrauben des Verbindungselements mit der Spülleisten ein Herausrutschen vermieden und somit die Reinigungsflotte zuverlässig in den Schlauch eingeleitet werden.

Die Gerüsteinheit kann gemäß einer Ausführungsform in einem zusammengesetzten Zustand L-förmig ausgeformt sein. Vorteilhafterweise kann durch die L-förmige Anordnung der Gerüsteinheit eine Ladefläche des Korbelements des Reinigungsgeräts effizient genutzt werden. Das bedeutet, dass die Gerüsteinheit platzsparend ausgeformt ist, sodass auch weiteres Reinigungsgut in das Korbelement eingeladen werden kann. Vorteilhafterweise kann die Gerüsteinheit aus einem Korbelement des Reinigungsgeräts je nach Anwendung eingesetzt oder entnommen werden. Durch die Form des Gerüstelements kann eine Beladungsfläche des Korbelements auch für weiteres Spülgut genutzt werden. Durch ein Gleichteil-Konzept kann weiterhin eine kostengünstige Fertigung erreicht werden. Zudem kann die Gerüsteinheit einfach erweitert werden, wodurch beispielsweise Kosten reduziert werden können.

Weiterhin kann das mindestens eine Halteelement H-förmig ausgeformt sein. Die Form des Halteelements können vorteilhafterweise Materialkosten reduziert werden, sodass die Aufnahmevorrichtung beispielsweise für einen Kunden preislich an Attraktivität gewinnen kann.

Die Aufnahmevorrichtung ist flexibel aus dem Reinigungsgerät entnehmbar und zusätzlich oder alternativ in das Reinigungsgerät einsetzbar. Beispielsweise kann die Aufnahmevorrichtung universell einsetzbar sein, sodass sie vorteilhafterweise auch für bereits vorhandene oder bestehende Reinigungsgeräte genutzt werden kann.

Es wird ferner ein Reinigungsgerät vorgestellt, das mindestens ein Korbelement zum Aufnehmen von Reinigungsgut und eine Aufnahmevorrichtung in einer zuvor genannten Variante aufweist.

Beispielsweise kann das Reinigungsgerät als ein Haushaltgerät beschrieben sein oder entsprechend im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Reinigungsgeräts mit einer Aufnahmevorrichtung gemäß einem Ausführungsbeispiel;
- Figur 2: eine schematische Darstellung eines Korbelements gemäß einem Ausführungsbeispiel für ein Reinigungsgerät;
- Figur 3: eine schematische Darstellung einer Gerüsteinheit zur Verwendung in einem Ausführungsbeispiel einer Aufnahmevorrichtung;
- Figur 4: eine schematische Darstellung eines Einsetzelements zur Verwendung in einem Ausführungsbeispiel einer Aufnahmevorrichtung;
- Figur 5: eine schematische Darstellung eines Einsetzelements zur Verwendung in einem Ausführungsbeispiel einer Aufnahmevorrichtung;
- Figur 6: eine schematische Darstellung einer Gerüsteinheit zur Verwendung in einem Ausführungsbeispiel einer Aufnahmevorrichtung;
- Figur 7: eine schematische Darstellung eines Korbelements mit einem Ausführungsbeispiel einer Aufnahmevorrichtung für ein Reinigungsgerät;
- Figur 8: eine schematische Darstellung eines Verbindungselements zur Verwendung in einem Ausführungsbeispiel einer Aufnahmevorrichtung;
- Figur 9: eine schematische Seitendarstellung einer Gerüsteinheit zur Verwendung in einem Ausführungsbeispiel einer Aufnahmevorrichtung;
- Figur 10: eine schematische Darstellung eines Erweiterungselements zur Verwendung in einem Ausführungsbeispiel einer Aufnahmevorrichtung;
- Figur 11: eine schematische Darstellung eines Erweiterungselements zur Verwendung in einem Ausführungsbeispiel einer Aufnahmevorrichtung;
- Figur 12: eine schematische Darstellung eines Ausführungsbeispiels einer Aufnahmevorrichtung;
- Figur 13: eine schematische Darstellung eines Ausführungsbeispiels einer Aufnahmevorrichtung; und
- Figur 14: eine schematische Darstellung eines Ausführungsbeispiel eines Korbelements mit einer Aufnahmevorrichtung für ein Reinigungsgerät.

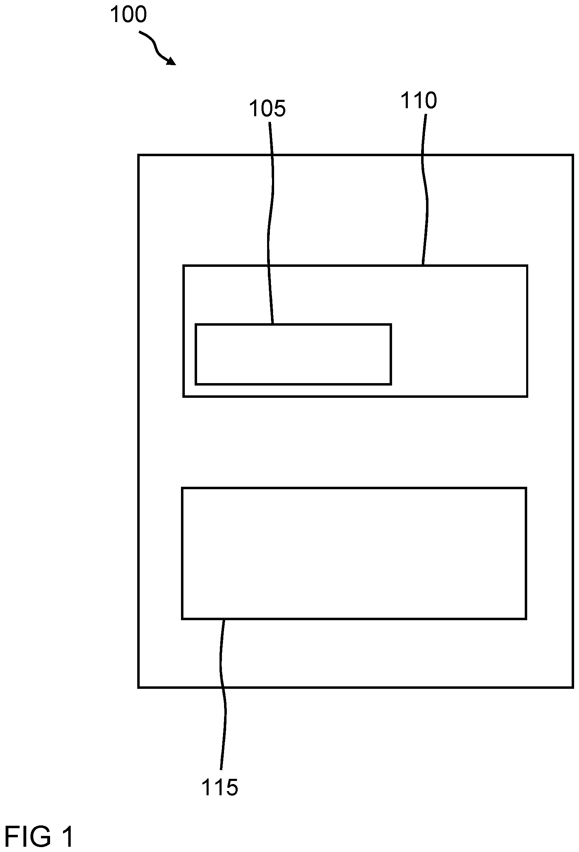

Figur 1 zeigt eine schematische Darstellung eines Reinigungsgeräts 100 mit einer Aufnahmevorrichtung 105 gemäß einem Ausführungsbeispiel. Die Aufnahmevorrichtung 105 wird dabei in mindestens einer der nachfolgenden Figuren näher beschrieben. Das Reinigungsgerät 100 weist weiterhin mindestens ein Korbelement 110 auf, das ausgebildet ist, um zu reinigendes Reinigungsgut aufzunehmen. Das Reinigungsgerät 100 weist insbesondere das Korbelement 110 als einen Oberkorb auf sowie lediglich optional zusätzlich dazu ein weiteres Korbelement 115, das beispielsweise als Unterkorb ausgeformt ist. Gemäß diesem Ausführungsbeispiel ist das Reinigungsgerät 100 als ein Spülgerät realisiert, das beispielsweise zu privaten Zwecken oder aber als professionelles Gerät, beispielsweise im medizinischen Bereich, genutzt wird. Die Aufnahmevorrichtung 105 ist gemäß diesem Ausführungsbeispiel in das Korbelement 110 eingesetzt, ist jedoch auch in das weitere Korbelement 115 einsetzbar. Die Aufnahmevorrichtung 105 ist ausgeformt, um mindestens einen Schlauch oder ein Schlauchset, wie es beispielsweise in der Zahnmedizin eingesetzt wird, für einen Reinigungsvorgang zu aufzunehmen. Dabei wird der Schlauch abschüssig in der Aufnahmevorrichtung 105 angeordnet, sodass Reste einer Reinigungsflotte nach dem Reinigen des Schlauchs abfließen können. Die Aufnahmevorrichtung 105 gemäß diesem Ausführungsbeispiel ist je nach Bedarf in das Reinigungsgerät 100 einsetzbar oder auch aus dem Reinigungsgerät 100 herausnehmbar ausgeformt. Die Aufnahmevorrichtung 105 ist weiterhin als eine modulare Aufnahmevorrichtung 105 ausgeformt, die eine Gerüsteinheit mit mindestens einem Gerüstelement und mindestens einem Halteelement, mindestens ein Einsetzelement sowie mindestens ein Verbindungselement aufweist. Die einzelnen Teile der Aufnahmevorrichtung 105 werden jedoch mindestens in einer der nachfolgenden Figuren näher erläutert.

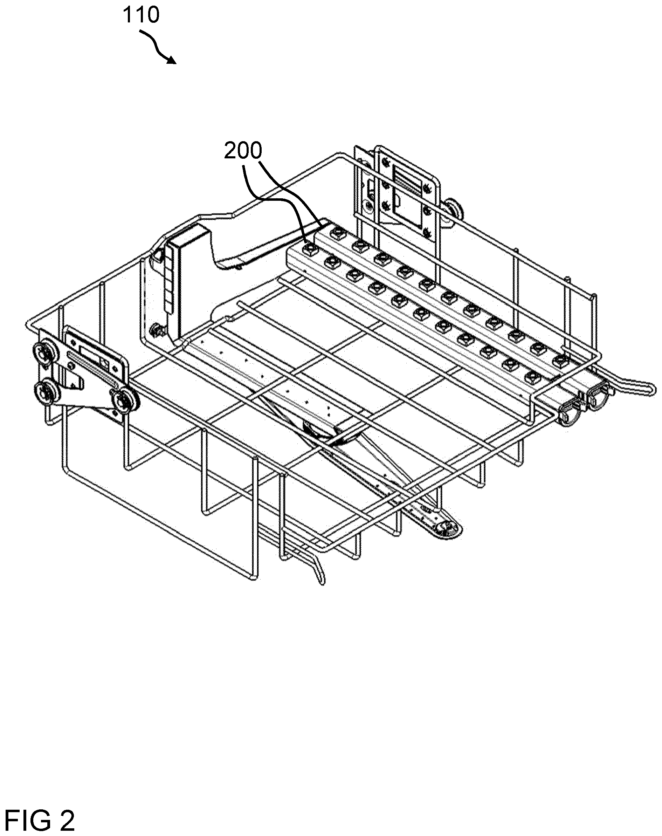

Figur 2 zeigt eine schematische Darstellung eines Korbelements 110 gemäß einem Ausführungsbeispiel für ein Reinigungsgerät. Das Korbelement 110 entspricht beispielsweise dem in Figur 1 beschriebenen Korbelement 110 und ist beispielsweise als ein Oberkorb ausgeformt. Das Korbelement 110 weist dabei mindestens eine Spülleiste 200, insbesondere zwei Spülleisten 200 auf, die beispielsweise ausgebildet sind, um eine Reinigungsflotte in die Aufnahmevorrichtung einzuleiten. Das bedeutet, dass die Spülleisten 200 gemäß diesem Ausführungsbeispiel als Anschlussschnittstellen ausgeformt sind. Gemäß diesem Ausführungsbeispiel ist die Aufnahmevorrichtung nicht in das Korbelement 110 eingesetzt.

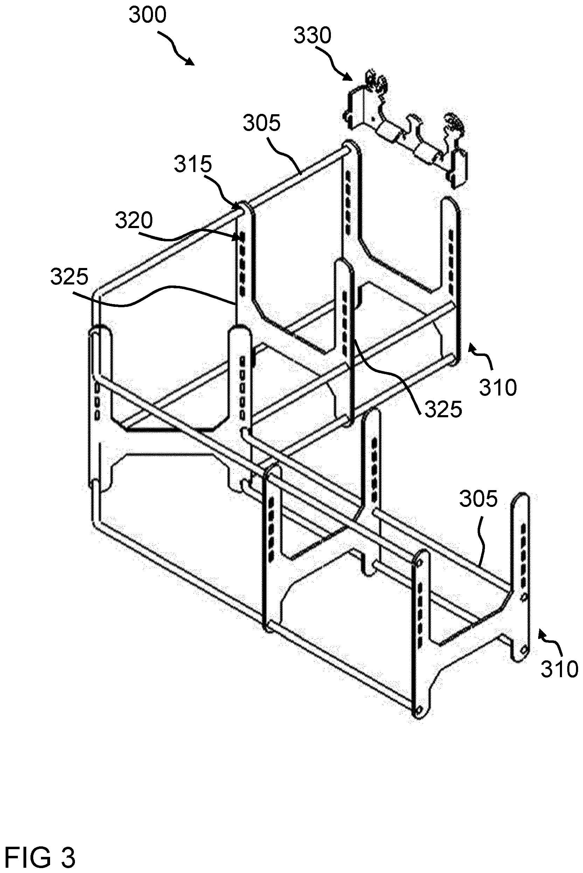

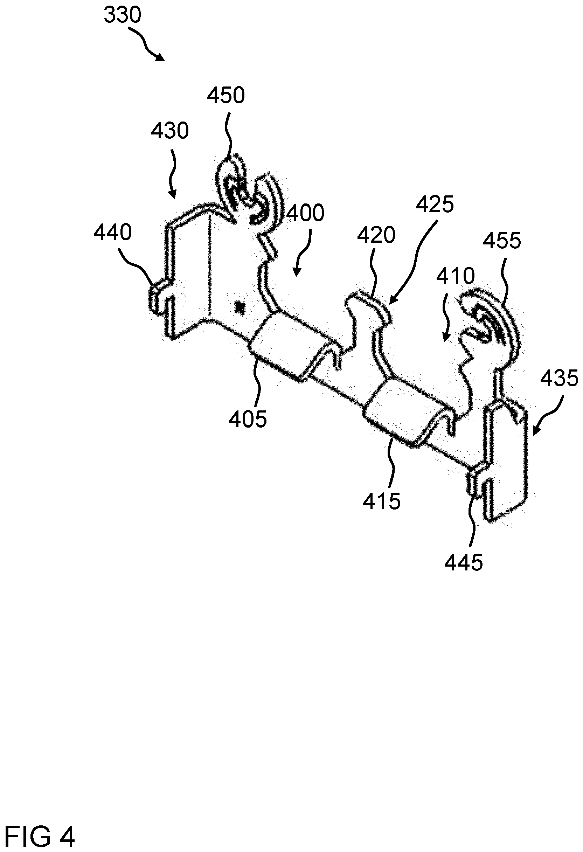

Figur 3 zeigt eine schematische Darstellung einer Gerüsteinheit 300 zur Verwendung in einem Ausführungsbeispiel einer Aufnahmevorrichtung. Die Gerüsteinheit 300 ist dabei als Teil einer Aufnahmevorrichtung ausgeformt, wie sie beispielsweise in Figur 1 erwähnt wurde und wie sie in mindestens einer der nachfolgenden Figuren beschrieben ist. Die Gerüsteinheit 300 weist dabei mindestens ein Gerüstelement 305 und mindestens ein mit dem Gerüstelement 305 zusammengesetztes und/oder zusammensetzbares Halteelement 310 auf. Das Gerüstelement 305 ist dabei beispielsweise als eine Stange ausgeformt, die gemäß diesem Ausführungsbeispiel durch eine Öffnung 315 in dem Halteelement 310 gesteckt ist. Gemäß diesem Ausführungsbeispiel weist die Gerüsteinheit 300 eine Mehrzahl von Gerüstelementen 305 sowie eine Mehrzahl von Halteelementen 310 auf, die gemäß diesem Ausführungsbeispiel in zusammengesetzten Zustand als Gerüst dargestellt sind. Die zusammengesetzte Gerüsteinheit 300 ist dabei gemäß diesem Ausführungsbeispiel L-förmig realisiert, sodass sie beispielsweise in ein Korbelement eines Reinigungsgeräts einsetzbar ist, wie es beispielsweise in einer der Figuren 1 bis 2 beschrieben wurde. Die Halteelemente 310 sind dabei H-förmig realisiert und sind lediglich optional in regelmäßigen Abständen zueinander entlang dem mindestens einen Gerüstelement 305 angeordnet. Gemäß diesem Ausführungsbeispiel weist jedes der Halteelemente 310 eine Mehrzahl von Durchgangsöffnungen 320 auf. Die Durchgangsöffnungen 320 sind dabei in einer Reihe übereinander und/oder an gegenüberliegenden Schenkeln 325 des Halteelements 310 angeordnet. Zusätzlich ist gemäß diesem Ausführungsbeispiel ein Einsetzelement 330 dargestellt, das in das Halteelement 310 einsetzbar, einschiebbar, einklipsbar oder einrastbar ist. Das Einsetzelement 330 ist als Teil der Aufnahmevorrichtung ausgeformt und wird in Figur 4 näher beschrieben. Gemäß diesem Ausführungsbeispiel wird pro Halteelement 310 ein Einsetzelement 330 eingesetzt.

Figur 4 zeigt eine schematische Darstellung eines Einsetzelements 330 zur Verwendung in einem Ausführungsbeispiel einer Aufnahmevorrichtung. Das Einsetzelement 330 entspricht beispielsweise dem in Figur 3 erwähnten Einsetzelements 330. Das Einsetzelement 330 ist dabei als Teil einer modularen Aufnahmevorrichtung realisiert oder realisierbar, die für ein Reinigungsgerät verwendet wird, wie es beispielsweise in Figur 1 beschrieben wurde. Das Einsetzelement 330 weist dazu einen Aufnahmebereich 400 auf, der ausgeformt ist, um den Schlauch aufzunehmen. Der Aufnahmebereich 400 ist beispielsweise als ein Ausschnitt ausgeformt. Weiterhin weist das Einsetzelement 330 gemäß diesem Ausführungsbeispiel an den Aufnahmebereich 400 angrenzend eine Stütznase 405 auf. Die Stütznase 405 ist gemäß diesem Ausführungsbeispiel einteilig mit dem Einsetzelement 330 ausgeformt. Zusätzlich oder optional ist die Stütznase 405 durch einen umgebogenen Stanzausschnitt des Einsetzelements 330 ausgeformt. Gemäß diesem Ausführungsbeispiel ist die Stütznase 405 als umgebogener Stanzausschnitt um beispielsweise mehr als 90° zu dem Einsetzelement 330 umgebogen. Ferner weist das Einsetzelement 330 mindestens einen weiteren Aufnahmebereich 410 auf, der ausgebildet ist, um einen weiteren Schlauch zu halten. Der weitere Aufnahmebereich 410 entspricht gemäß diesem Ausführungsbeispiel dem Aufnahmebereich 400 in seiner Form. Das Einsetzelement 330 weist analog zu dem Aufnahmebereich 400 ebenfalls eine an den weiteren Aufnahmebereich 410 angrenzende weitere Stütznase 415 auf. Die Stütznasen 405, 415 sind beispielsweise gleichartig ausgeformt. Getrennt werden die beiden Aufnahmebereiche 400, 410 gemäß diesem Ausführungsbeispiel durch einen Trennsteg 420, der zwischen den Aufnahmebereichen 400, 410 angeordnet ist. Der Trennsteg 420 ist dabei mindestens teilweise pilzartig ausgeformt. Das bedeutet, dass der Trennsteg 420 gemäß diesem Ausführungsbeispiel in Richtung eines freien Endes 425 des Trennstegs 420 aufgeweitet ausgeformt ist. Gemäß diesem Ausführungsbeispiel liegen die Aufnahmebereiche 400, 410 sowie der Trennsteg 420 auf einer Ebene. Weiterhin weist das Einsetzelement 330 gemäß diesem Ausführungsbeispiel einen ersten Randbereich 430 auf, der benachbart zu dem Aufnahmebereich 400 angeordnet ist. Der erste Randbereich 430 ist dabei quer zu dem Aufnahmebereich 400 ausgerichtet, beispielsweise umgebogen. Analog dazu weist das Einsetzelement 330 benachbart zu dem weiteren Aufnahmebereich 400 einen zweiten Randbereich 435 auf. Gemäß diesem Ausführungsbeispiel sind die Randbereiche 430, 435 parallel zueinander ausgerichtet. Das Einsetzelement 330 weist dabei im ersten Randbereich 430 ein erstes Einhakelement 440 und im zweiten Randbereich 435 ein zweites Einhakelement 445 auf. Die Einhakelemente 440, 445 sind dabei ausgebildet, um das Einsetzelement 330 in das Halteelement, genauer gesagt in die Durchgangsöffnungen des Halteelements einzuhaken, wie sie beispielsweise in Figur 3 beschrieben wurden. Die Einhakelemente 440, 445 sind parallel zueinander angeordnet. Weiterhin liegt das erste Einhakelement 440 auf einer Ebene mit dem ersten Randbereich 430 und das zweite Einhakelement 445 liegt auf einer Ebene mit dem zweiten Randbereich 435.

Gemäß diesem Ausführungsbeispiel weist das Einsetzelement 330 zusätzlich mindestens ein Einklipselement 450 auf, das ausgebildet ist, um ein Erweiterungselement an dem Einsetzelement zu befestigen. Dem Einklipselement 450 gegenüberliegend weist das Einsetzelement 330 ein weiteres Einklipselement 455 auf. Die Einklipselemente 450, 455 sind dabei gemäß diesem Ausführungsbeispiel C-förmig realisiert. Weiterhin zeigt lediglich optional eine Öffnung des weiteren Einklipselements 455 in Richtung des Einklipselements 450. Das bedeutet, dass die Öffnungen der Einklipselemente 450, 455 in unterschiedliche Richtungen zeigen. In einem alternativen Ausführungsbeispiel ist es denkbar, dass die Öffnungen in die gleiche Richtung zeigen.

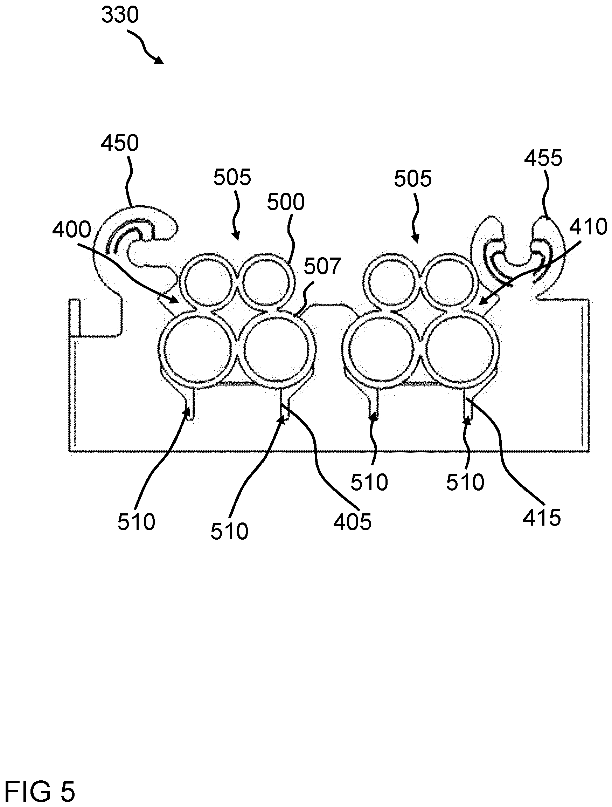

Figur 5 zeigt eine schematische Darstellung eines Einsetzelements 330 zur Verwendung in einem Ausführungsbeispiel einer Aufnahmevorrichtung. Das Einsetzelement 330 entspricht beispielsweise dem in Figur 4 beschriebenen Einsetzelement 330 und weicht lediglich in der Darstellungsperspektive ab. Zudem ist gemäß diesem Ausführungsbeispiel mindestens ein Schlauch 500 eines Schlauchsets 505 und insbesondere mindestens ein weiterer Schlauch 507 dargestellt. Jeweils ein Schlauchset 505 ist dabei in einem der Aufnahmebereiche 400, 410 angeordnet. Gemäß diesem Ausführungsbeispiel weist das Einsetzelement 330 im Bereich der Stützelemente 405, 415 jeweils zwei Einschnitte 510 auf. Die Einschnitte 510 erlauben eine besonders einfache Herstellung der Stütznasen 405, 415 durch einfaches Umbiegen. Darüber hinaus bewirkt diese Ausbildung eine gewisse Flexibiltät bei der Halterung der aufgenommenen Schläuche. Gemäß diesem Ausführungsbeispiel weist jedes der Schlauchsets 505 lediglich beispielhaft vier Schläuche auf, die lediglich optional als zwei Zweierverbünde mit jeweils den zwei Schläuchen 500, 507 ausgeformt sind. Die Schläuche 500, 507 innerhalb eines Zweierverbunds weisen dabei gemäß diesem Ausführungsbeispiel unterschiedliche Durchmesser auf. Genauer gesagt weist der weitere Schlauch 507 einen größeren Durchmesser auf als der Schlauch 500.

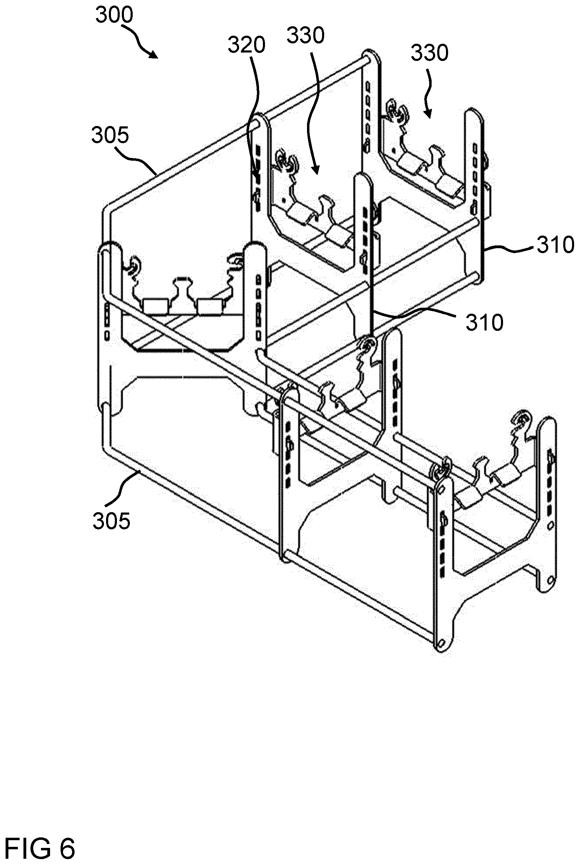

Figur 6 zeigt eine schematische Darstellung einer Gerüsteinheit 300 zur Verwendung in einem Ausführungsbeispiel einer Aufnahmevorrichtung. Die dargestellte Gerüsteinheit 300 entspricht beispielsweise der in Figur 3 beschriebenen Gerüsteinheit 300. Gemäß diesem Ausführungsbeispiel ist die Gerüsteinheit 300 in zusammengesetztem Zustand und mit eingesetzten Einsetzelementen 330 dargestellt. Jedes der Einsetzelemente 330 ist dabei jeweils in ein Halteelement 310 eingesetzt. Weiterhin ist jedes der Einsetzelemente 330 auf einer anderen Höhenposition des Halteelements 310 eingesetzt, beispielsweise aufsteigend, sodass ein Schlauch beispielsweise an einem Ende tiefer liegt als an einem anderen Ende. Das bedeutet anders ausgedrückt, dass jedes der Einsetzelemente 330 jeweils in einer gegenüber einem vorigen Halteelement 310 höher angeordneten Durchgangsöffnung 320 des Halteelements 310 eingehakt ist. Dadurch wird beispielsweise eine schräge Anordnung des Schlauchs oder Schlauchsets ermöglicht, sodass ein vollständiges Ablaufen einer Reinigungsflüssigkeit aus dem Schlauch ermöglicht wird.

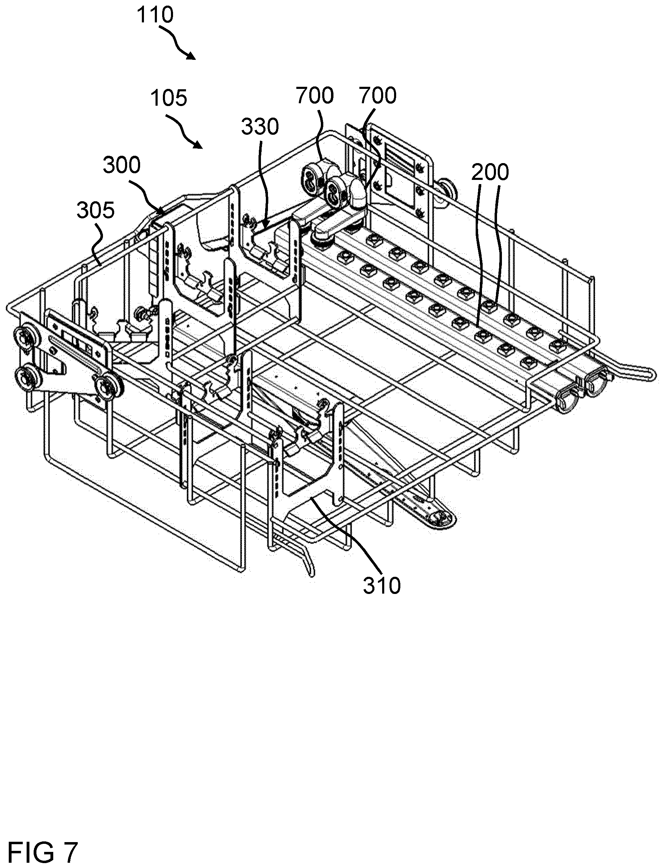

Figur 7 zeigt eine schematische Darstellung eines Korbelements 110 mit einem Ausführungsbeispiel einer Aufnahmevorrichtung 105 für ein Reinigungsgerät. Das Korbelement 110 entspricht oder ähnelt beispielsweise dem in Figur 2 beschriebenen Korbelement 110. Ebenso ist das Korbelement 110 für ein Reinigungsgerät einsetzbar, wie es beispielsweise in Figur 1 beschrieben wurde. Weiterhin ist gemäß diesem Ausführungsbeispiel die Aufnahmevorrichtung 105 in das Korbelement 110 eingesetzt. Die Aufnahmevorrichtung 105 ist ausgebildet, um einen Schlauch aufzunehmen und weist eine Gerüsteinheit 300 mit dem Gerüstelement 305 und dem mindestens einen mit dem Gerüstelement 305 zusammengesetzten und/oder zusammensetzbaren Halteelement 310 auf, wie sie beispielsweise in den Figuren 3 und/oder 6 beschrieben wurden. Zusätzlich weist die Aufnahmevorrichtung 105 das mindestens eine in das Halteelement 310 einsetzbare Einsetzelement 330 mit dem Aufnahmebereich auf, der ausgeformt ist, um den Schlauch aufzunehmen. Weiterhin weist die Aufnahmevorrichtung 105 mindestens ein Verbindungselement 700 auf, das ausgeformt ist, um den Schlauch mit der Spülleiste 200 des Reinigungsgeräts zu verbinden und eine Reinigungsflüssigkeit in den Schlauch einzuleiten. Hierzu kann das Verbindungselement 700 etwa mit der Spülleiste 200 verschraubt sein. Weiterhin weist die Aufnahmevorrichtung 105 gemäß diesem Ausführungsbeispiel ein weiteres Verbindungselement auf, das dem Verbindungselement 700 entspricht. Beispielsweise sind für ein Schlauchset, wie es beispielsweise in Figur 5 dargestellt ist, zwei Verbindungselemente 700 mit der Spülleiste 200 verbunden. Die Verbindungselemente 700 weisen dabei eine Adapterfunktion auf. Insbesondere sind die Verbindungselemente 700 mit zwei parallel zueinander angeordneten Spülleisten 200 verbunden.

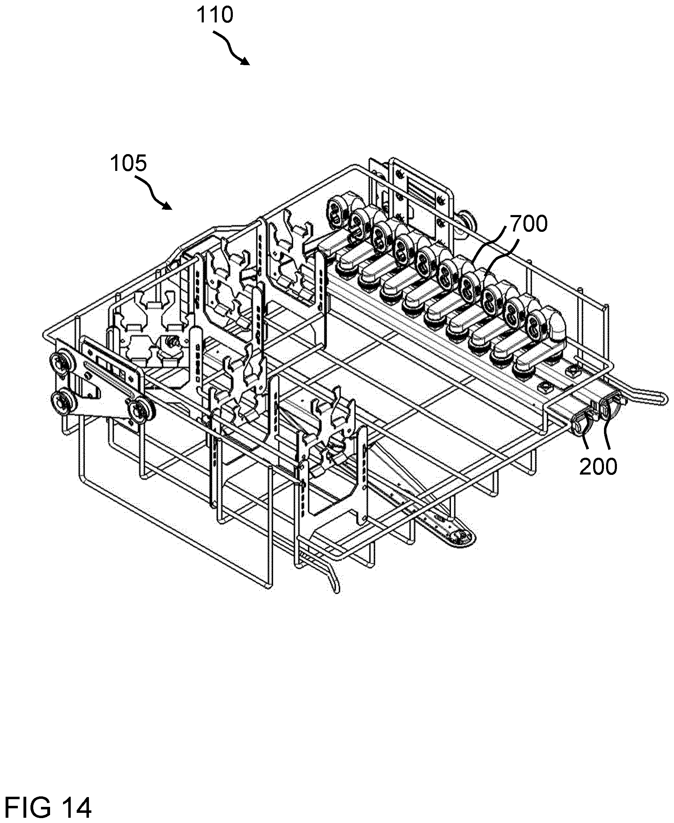

Gemäß diesem Ausführungsbeispiel wird die Aufnahme und Adaption von Schläuchen in einem bestehenden Beladungsträger, beispielsweise ein Korbelement 110, auf einer Spülebene modular ermöglicht. Ein Kunde muss keinen speziellen Beladungsträger für diesen Anwendungsfall kaufen, sondern kann einen Bestehenden mit einer neuen Funktion ausstatten. Hierfür werden das Verbindungselement 700 und die Gerüsteinheit 300 samt Einsetzelement 330, zusammengefasst die Aufnahmevorrichtung 105, benötigt. Das Verbindungselement 700 ist beispielsweise auf der bestehenden Spülleiste 200 aufgeschraubt. Die Gerüsteinheit 300, die beispielsweise auch als Einsatz bezeichnet ist, wird auf die Beladungsfläche des Korbelements 110 gestellt und füllt diese nicht vollständig aus, sodass beispielsweise noch weiteres Reinigungsgut auf der Beladungsfläche Platz findet. Die Aufnahmevorrichtung 105 kann gemäß diesem Ausführungsbeispiel zwei Schlauchsets aufnehmen. Für weitere Schläuche kann die Aufnahmevorrichtung 105 erweitert und die Anzahl von Verbindungselementen 700 erhöht werden, wie es beispielsweise in Figur 14 dargestellt ist.

Die Gerüsteinheit 300 ist in anderen Worten beispielsweise L-förmig realisiert und ist lediglich optional aus auch als Bleche bezeichneten Halteelemente 310 und gebogenen Drähten, die als Gerüstelemente 305 beschrieben sind, zu einer Baugruppe verschweißt. In den Halteelementen 310 sind beispielsweise übereinander in gleichen Abständen angeordnete Durchgangsöffnungen 320 zu sehen. In diese Öffnungen 320 werden die Einsetzelemente 330, die auch als Schlauchhalterung bezeichnet sind, beispielsweise mittels Einhakelementen eingehakt. Dabei wird beginnend von den Verbindungselementen die unterste Durchgangsöffnung 320 für ein erstes der Einsetzelemente 330 verwendet. Alle weiteren Einsetzelemente 330 werden steigend in die nächsthöhere der Durchgangsöffnungen 320 eingehakt, sodass sich eine steigende Anordnung ergibt. Die Einsetzelemente 330 weisen dabei eine Kontur auf, die durch einen Trennsteg 420 eine getrennte Aufnahme von zwei Schlauchsets ermöglicht. Für weitere Schlauchsets werden beispielsweise Erweiterungselemente 1000 in die vorhandenen Einsetzelemente 330 eingesetzt. Hierfür weist jedes der Einsetzelemente 330 eine als Einklipselemente 450, 455 beschriebene Aufnahmekontur auf, in welche die Erweiterungselemente 1000 einrasten und über eine Achse drehbar sind. Für eine Beladung mit den ersten beiden Schlauchsets werden die Erweiterungselemente 1000 beispielsweise nach innen geschwenkt, um die Aufnahmebereiche der Einsetzelemente 330 freizugeben. Anschließend werden die Erweiterungselemente 1000 zurückgeschwenkt und befinden sich so oberhalb der ersten zwei Schlauchsets. Jedes der Erweiterungselemente 1000 weist eine Kontur für die getrennte Aufnahme von drei Schlauchsets auf.

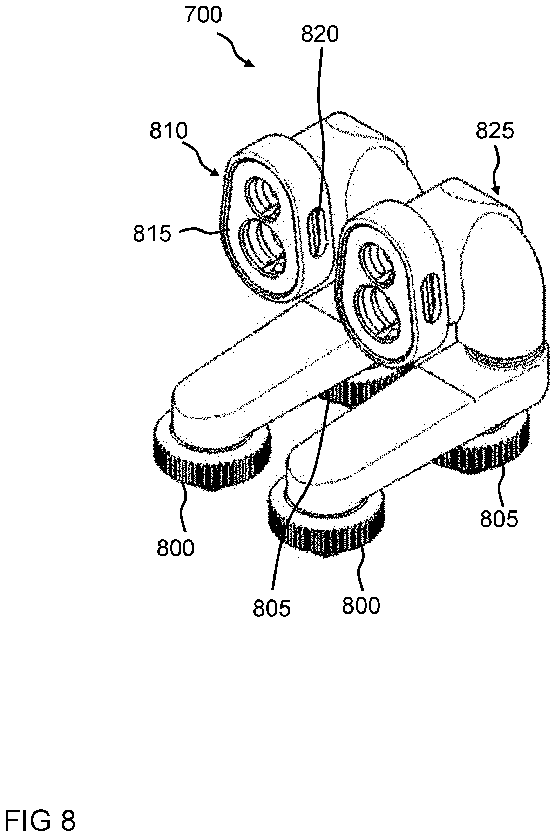

Figur 8 zeigt eine schematische Darstellung eines Verbindungselements 700 zur Verwendung in einem Ausführungsbeispiel einer Aufnahmevorrichtung. Die in Figur 8 dargestellten Verbindungselemente 700 entsprechen beispielsweise den in Figur 7 beschriebenen Verbindungselementen 700. Die Verbindungselemente 700 weisen dabei jeweils mindestens eine Anschlussstelle 800 auf, mittels der das Verbindungselement 700 mit der Spülleiste des Korbelements verbunden, beispielsweise verschraubt wird. Insbesondere sind die Verbindungselemente 700 mit beiden Spülleisten verbindbar, sodass ausreichend Reinigungsflüssigkeit in alle zu reinigenden Schläuche geleitet werden kann. Das bedeutet, dass ein Verbindungselement 700 jeweils mindestens eine weitere Anschlussstelle 805 aufweist, das auf einer Linie mit der Anschlussstelle 800 liegt. An einem den Anschlussstellen 800, 805 abgewandten Ende des Verbindungselements 700 weist das Verbindungselement 700 eine Verbindungsöffnung 810 auf, in die beispielsweise ein Dichtelement 815 einsetzbar ist. Das Dichtelement 815 ist lediglich optional austauschbar, sodass eine Vielzahl unterschiedlicher Schlaucharten mit dem Verbindungselement 700 verbindbar sind. Gemäß diesem Ausführungsbeispiel weist das Dichtelement 810 zwei unterschiedlich große Öffnungen auf, die beispielsweise mit einem Zweierverbund von Schläuchen, beziehungsweise mit einem Schlauchset koppelbar sind, wie er beispielsweise in Figur 5 beschrieben wurde.

Weiterhin optional ist die Verbindungsöffnung 810 in eine den Anschlussstellen 800, 805 abgewandte Richtung sich verjüngend dargestellt. An einer Mantelfläche der Verbindungsöffnung 810 weist das Verbindungselement 700 ferner mindestens eine oval ausgeformte Sicherungsöffnung 820 auf, die beispielsweise ausgebildet ist, um das Dichtelement 815 in der Verbindungsöffnung 810 zu sichern, es etwa einzurasten.

Das Verbindungselement 700 weist beispielsweise eine drehbare Aufnahme auf, mit der die Ausrichtung der Schläuche zur Gerüsteinheit erfolgt. In der Aufnahme, die gemäß diesem Ausführungsbeispiel als Verbindungsöffnung 810 beschrieben ist, befindet sich eine elastische auch als Dichtelement 815 beschriebene Dichtung. Die Kontur des Dichtelements 815 ist derart gestaltet, dass unterschiedliche Durchmesser und Abstände des starren Doppelstutzens 825 aufgenommen werden können.

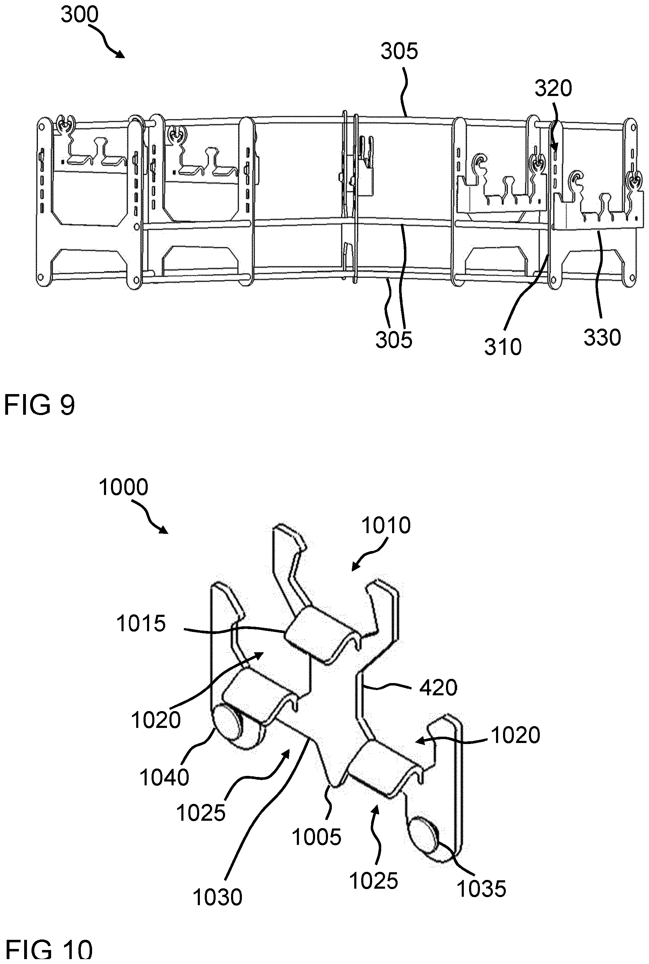

Figur 9 zeigt eine schematische Seitendarstellung einer Gerüsteinheit 300 zur Verwendung in einem Ausführungsbeispiel einer Aufnahmevorrichtung. Die gemäß diesem Ausführungsbeispiel dargestellte Gerüsteinheit 300 entspricht beispielsweise der in einer der Figuren 3, 6 oder 7 beschriebenen Gerüsteinheit 300. Auch weist die Gerüsteinheit 300 gemäß diesem Ausführungsbeispiel mindestens ein Einsetzelement 330 auf, wie es beispielsweise in mindestens einer der Figuren 3 bis 7 beschrieben wurde. Lediglich eine Darstellungsperspektive ist in Figur 9 unterschiedlich. Gemäß diesem Ausführungsbeispiel werden die Höhenpositionen der in die Halteelemente 310 eingesetzten Einsetzelemente 330 verdeutlicht, wobei beispielsweise an einem rechten äußeren Ende der Gerüsteinheit 300 das Einsetzelement 330 in einer untersten der Durchgangsöffnungen 320 eingesetzt ist. An einem dazu links benachbarten Halteelement 310 ist das Einsetzelement 330 beispielsweise eine Durchgangsöffnung 320 höher als bei dem äußersten Halteelement 310 angeordnet. Das bedeutet, dass die Einsetzelemente 330 gemäß diesem Ausführungsbeispiel von rechts nach links jeweils in einer höher positionierten Durchgangsöffnung 320 angeordnet sind als das jeweils vorige Einsetzelement 330, sodass eine lineare Steigung sichtbar ist. Umgekehrt, wenn die Einsetzelemente 330 von links nach rechts betrachtet werden, sind die Einsetzelemente 330 pro Halteelement 310 tiefer angeordnet als bei einem vorigen Halteelement 310. Durch diese Steigung wird beispielsweise erreicht, dass der mindestens eine Schlauch abschüssig angeordnet ist.

Figur 10 zeigt eine schematische Darstellung eines Erweiterungselements 1000 gemäß einem Ausführungsbeispiel für eine Aufnahmevorrichtung. Das Erweiterungselement 1000 ist beispielsweise in einer Aufnahmevorrichtung einsetzbar, wie sie beispielsweise in Figur 7 beschrieben wurde. Genauer gesagt ist das Erweiterungselement 1000 in ein Einsetzelement einsetzbar, wie es beispielsweise in mindestens einer der Figuren 3 bis 7 und/oder 9 beschrieben wurde. Das Erweiterungselement 1000 weist ebenfalls den Trennsteg 420 auf, der gemäß diesem Ausführungsbeispiel jedoch Y-förmig realisiert ist. Lediglich optional weist das Erweiterungselement 1000 zusätzlich einen weiteren Trennsteg 1005 auf, der pyramidenförmig ausgeformt ist und in eine dem Trennsteg 420 abgewandte Richtung zeigt. Weiterhin weist das Erweiterungselement 1000 mindestens einen Erweiterungsaufnahmebereich 1010 auf, der ausgebildet ist, um mindestens einen zusätzlichen Schlauch aufzunehmen. Der Erweiterungsaufnahmebereich 1010 ist dabei beispielsweise gleichartig ausgeformt, wie der mindestens eine Aufnahmebereich des in mindestens einer der vorangegangenen Figuren beschriebenen Einsetzelements. Das bedeutet, dass das Erweiterungselement 1000 angrenzend an den Erweiterungsaufnahmebereich 1010 eine Erweiterungsstütznase 1015 aufweist, die insbesondere einteilig mit dem Erweiterungselement 1000 ausgeformt ist, und/oder die durch einen umgebogenen Stanzausschnitt des Erweiterungselements 1000 ausgeformt ist. Insbesondere weist das Erweiterungselement 1000 gemäß diesem Ausführungsbeispiel mindestens zwei weitere Erweiterungsaufnahmebereiche 1020 auf, die gleichartig ausgeformt sind und auf einer Ebene liegen. Die weiteren Erweiterungsaufnahmebereiche 1020 werden dabei durch die Trennwand 420 voneinander getrennt, sodass der Erweiterungsaufnahmebereich 1010 auf mindestens einer Ebene versetzt zu den weiteren Erweiterungsaufnahmebereichen 1020 angeordnet ist. Weiterhin weist das Erweiterungselement 1000 gemäß diesem Ausführungsbeispiel zwei gleichartig ausgeformte Ausschnitte 1025 auf, die an den weiteren Trennsteg 1005 angrenzen. Die Ausschnitte 1025 sind gemäß diesem Ausführungsbeispiel an einer Unterkante 1030 des Erweiterungselements 1000 ausgeformt, sodass auch bei einem Zusammensetzen des Einsetzelements mit dem Erweiterungselement 1000 eine Aufnahme von zwei Schläuchen oder Schlauchsets in den dafür vorgesehenen Aufnahmebereichen des Einsetzelements möglich ist. Die Ausschnitte 1015 sind dabei gemäß diesem Ausführungsbeispiel unterhalb der weiteren Erweiterungsaufnahmebereiche 1020 angeordnet. Das bedeutet, dass die Ausschnitte 1025 auf einer Höhe mit den Aufnahmebereichen angeordnet sind und beispielsweise als eine Oberkante der Aufnahmebereiche des Einsetzelements fungieren.

Das Erweiterungselement 1000 weist gemäß diesem Ausführungsbeispiel mindestens eine Haltenoppe 1035 auf, die beispielsweise ausgebildet ist, um das Erweiterungselement 1000 mit dem Einsetzelement zu verbinden. Dazu wird die Haltenoppe 1035 optional in ein Einklipselement des Einsetzelements beispielsweise mittels Schwenken eingehakt. Insbesondere weist das Erweiterungselement 1000 eine weitere Haltenoppe 1040 auf, die an einer der Haltenoppe 1035 gegenüberliegenden Seite des Erweiterungselements 1000 angeordnet ist. Die Haltenoppen 1035, 1040 weisen beispielsweise einen Schaft und einen an den Schaft angrenzenden Kopf auf, wobei der Kopf beispielsweise knopfartig ausgeformt ist.

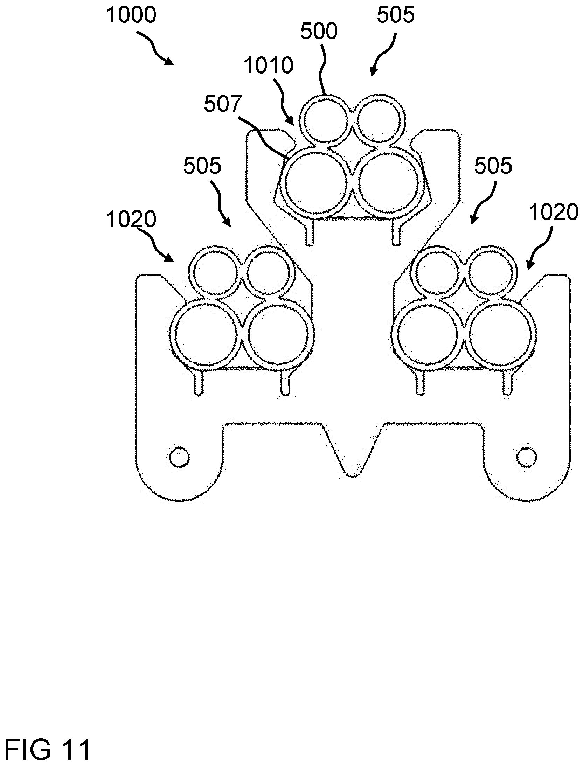

Figur 11 zeigt eine schematische Darstellung eines Erweiterungselements 1000 zur Verwendung in einem Ausführungsbeispiel einer Aufnahmevorrichtung. Das in Figur 11 dargestellte Erweiterungselement 1000 entspricht beispielsweise dem in Figur 10 beschriebenen Erweiterungselement 1000. Lediglich abweichend ist gemäß diesem Ausführungsbeispiel die Darstellungsperspektive. Zudem sind gemäß diesem Ausführungsbeispiel eine Mehrzahl von Schläuchen 500, 507 beziehungsweise drei Schlauchsets 505, in den Erweiterungsaufnahmebereichen 1010, 1020 angeordnet, die allesamt, wie auch die in Figur 5 dargestellten Aufnahmebereiche des Einsetzelements, Einschnitte zum Ausgleichen von wirkenden Kräften aufweisen.

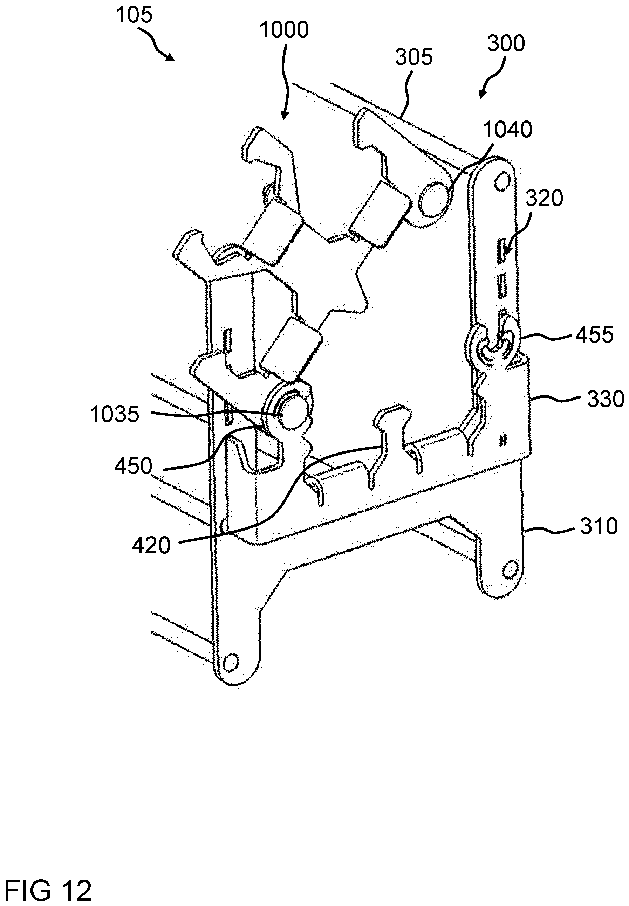

Figur 12 zeigt eine schematische Darstellung eines Ausführungsbeispiels einer Aufnahmevorrichtung 105. Die Aufnahmevorrichtung 105 weist gemäß diesem Ausführungsbeispiel das Erweiterungselement 1000 auf, wie es beispielsweise in mindestens einer der Figuren 10 bis 11 beschrieben wurde. Gemäß diesem Ausführungsbeispiel ist eine Haltenoppe 1035 des Erweiterungselements 1000 in ein Einklipselement 450 des Einsetzelements 330 eingeklipst und somit befestigt. Im Anschluss daran ist das Erweiterungselement 1000 schwenkbar, sodass die weitere Haltenoppe 1040 mit dem weiteren Einklipselement 455 verrastet oder durch Schwenken miteinander verrastbar sind. Die Einklipselemente 450, 455 sind dabei einander gegenüberliegend angeordnet und sind gemäß diesem Ausführungsbeispiel C-förmig realisiert.

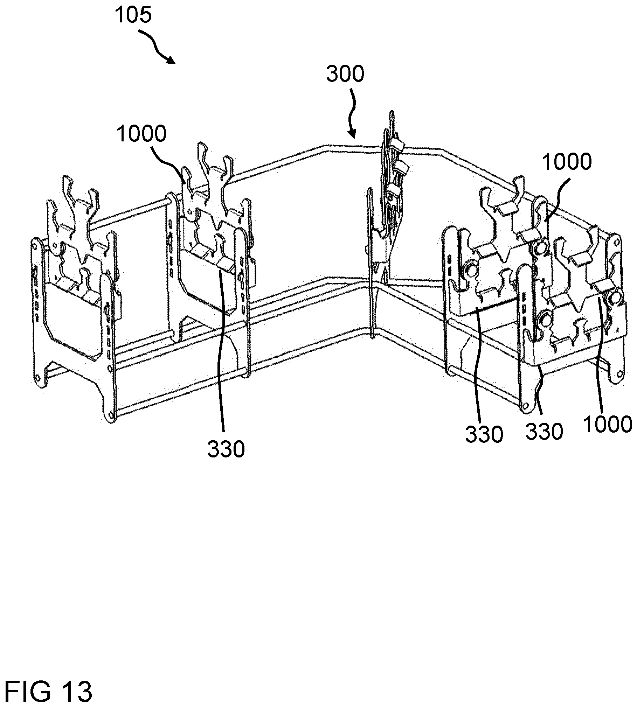

Figur 13 zeigt eine schematische Darstellung eines Ausführungsbeispiels einer Aufnahmevorrichtung 105. Die Aufnahmevorrichtung 105 entspricht oder ähnelt mindestens der in Figur 12 beschriebenen Aufnahmevorrichtung 105 und ist beispielsweise für ein Reinigungsgerät verwendbar, wie es beispielsweise in Figur 1 beschrieben wurde. Gemäß diesem Ausführungsbeispiel ist die Aufnahmevorrichtung 105 in zusammengebautem und erweitertem Zustand dargestellt, was bedeutet, dass an jedem der Einsetzelemente 330 jeweils ein Erweiterungselement 1000 angeordnet ist. Anders ausgedrückt ist gemäß diesem

Ausführungsbeispiel eine Gerüsteinheit 300, wie sie in mindestens einer der Figuren 3, 6, 7 und/oder 9 dargestellt und beschrieben wurde, erweitert dargestellt.

Figur 14 zeigt eine schematische Darstellung eines Ausführungsbeispiel eines Korbelements 110 mit einer Aufnahmevorrichtung 105 für ein Reinigungsgerät. Die in Figur 14 dargestellte Aufnahmevorrichtung 105 entspricht beispielsweise der in Figur 13 beschriebenen Aufnahmevorrichtung 105. Lediglich ist sie gemäß diesem Ausführungsbeispiel in das Korbelement 110 eingesetzt dargestellt, wie es beispielsweise in Figur 2 und/oder 7 beschrieben wurde. Da gemäß diesem Ausführungsbeispiel fünf Schlauchsets gleichzeitig in die Aufnahmevorrichtung 105 platzierbar sind und pro Schlauchset zwei Verbindungselemente 700 für einen Reinigungsvorgang gefordert sind, sind gemäß diesem Ausführungsbeispiel insgesamt zehn gleichartige Verbindungselemente 700 in dem Korbelement 110 angeordnet und mit der mindestens einen Spülleiste 200 verbunden.

In anderen Worten ausgedrückt ermöglicht der hier vorgestellte Ansatz eine Aufnahme und Adaption von Schläuchen in einem bestehenden Beladungsträger für eine modulare Aufbereitung von beispielsweise Lachgasschläuchen. Für die maschinelle Reinigung und Desinfektion von medizinischen Schläuchen beispielsweise für Lachgasanwendungen werden diese im Beladungsträger, der hier als Korbelement 110 beschrieben ist, eines Reinigungs- und Desinfektionsgeräts (RDG) adaptiert, gehalten und positioniert. Dabei ist es für die Reinigung wichtig, dass die Schläuche steigend verlegt werden, damit sich keine Spülflotte ansammelt und damit die Spülflotte frei ausläuft. Weiterhin sind die Schläuche universell mit einem Adapter, der als Verbindungselement 700 beschrieben ist, angeschlossen oder anschließbar. Die Adaption der Schläuche erfolgt beispielsweise über das mindestens eine Verbindungselement 700, das auf zwei Spülleisten 200 geschraubt ist, um einen ausreichend großen Volumenstrom zur Verfügung zu stellen.

In anderen Worten ausgedrückt werden die Schläuche auf einer Ebene aufbereitet, um weiterhin beide Beladungsebenen im Reinigungsgerät zu nutzen. Eine Programmführung im Reinigungsgerät ist dabei lediglich optional derart ausgelegt, dass ein stabiler Pumpendruck gewährleistet wird und die Schläuche gemeinsam mit anderem Reinigungsgut in einer Charge aufbereitbar sind. Damit werden Ressourcen geschont, wie beispielsweise ein zusätzlicher Beladungsträger, Wasser und/oder eine für den Reinigungsvorgang genutzte Energiemenge.

## Patentansprüche

1. Aufnahmevorrichtung (105) für ein Reinigungsgerät (100) zum Einsetzen in ein Korbelement (110) des Reinigungsgeräts (100), wobei die Aufnahmevorrichtung (105) zum Aufnehmen mindestens eines Schlauchs (500) dient und die folgenden Merkmale aufweist:
- mehrere Halteelemente (310), die jeweils eine Mehrzahl von Durchgangsöffnungen (320) aufweisen;
- mehrere, jeweils in eines der Halteelemente (310) einsetzbare Einsetzelemente (330), die jeweils einen Aufnahmebereich (400) aufweisen, der ausgeformt ist, um den Schlauch (500) aufzunehmen;
- mindestens ein Verbindungselement (700), das ausgeformt ist, um den Schlauch (500) mit einer Spülleiste (200) des Reinigungsgeräts (100) zu verbinden, um eine Reinigungsflüssigkeit in den Schlauch (500) einzuleiten,
**dadurch gekennzeichnet, dass**
die Aufnahmeeinrichtung (105) eine aus mehreren Gerüstelementen (305) und den mehreren mit den Gerüstelementen (305) zusammengesetzten Halteelementen (310) ausgeformte Gerüsteinheit (300) aufweist,
wobei die Einsetzelemente (330) jeweils in einem ersten Randbereich (430) ein erstes Einhakelement (440) und in einem zweiten Randbereich (435) ein zweites Einhakelement (445) aufweisen, die ausgebildet sind, um das jeweilige Einsetzelement (330) in eines der Halteelemente (310) einzuhaken,
und wobei mehrere Durchgangsöffnungen (320) eines jeweiligen Halteelements (310) jeweils in einer Reihe auf gegenüberliegenden Schenkeln (325) des Halteelements (310) zur Aufnahme des ersten) Einhakelements (440) und des zweiten Einhakelements (445) angeordnet sind.

2. Aufnahmevorrichtung (105) gemäß Anspruch 1, wobei das Einsetzelement (330) an den Aufnahmebereich (400) angrenzend eine Stütznase (405) aufweist, insbesondere wobei die Stütznase (405) einteilig mit dem Einsetzelement (330) ausgeformt ist, und/oder wobei die Stütznase (405) durch einen umgebogenen Stanzausschnitt des Einsetzelements (330) ausgeformt ist.

3. Aufnahmevorrichtung (105) gemäß einem der vorangegangenen Ansprüche, wobei das Einsetzelement (330) mindestens einen weiteren Aufnahmebereich (410) aufweist, der ausgebildet ist, um einen weiteren Schlauch (507) zu halten.

4. Aufnahmevorrichtung (105) gemäß Anspruch 3, wobei das Einsetzelement (330) zwischen dem Aufnahmebereich (400) und dem mindestens einen weiteren Aufnahmebereich (410) einen Trennsteg (420) zum Trennen der Aufnahmebereiche (400, 410) aufweist.

5. Aufnahmevorrichtung (105) gemäß Anspruch 4, wobei der Trennsteg (420) Y-förmig ausgeformt ist.

6. Aufnahmevorrichtung (105) gemäß einem der vorangegangenen Ansprüche, mit einem Erweiterungselement (1000), das in das Einsetzelement (330) einsetzbar ist, wobei das Erweiterungselement (1000) mindestens einen Erweiterungsaufnahmebereich (1010) aufweist, der ausgebildet ist, um mindestens einen zusätzlichen Schlauch zu halten.

7. Aufnahmevorrichtung (105) gemäß Anspruch 6, wobei das Einsetzelement (330) mindestens ein Einklipselement (450, 455) zum Befestigen des Erweiterungselements (1000) an dem Einsetzelement (330) aufweist.

8. Aufnahmevorrichtung (105) gemäß einem der vorangegangenen Ansprüche, wobei das Verbindungselement (700) mit der Spülleiste (200) verbunden, insbesondere verschraubt ist.

9. Aufnahmevorrichtung (105) gemäß einem der vorangegangenen Ansprüche, wobei die Gerüsteinheit (300) in einem zusammengesetzten Zustand L-förmig ausgeformt ist.

10. Aufnahmevorrichtung (105) gemäß einem der vorangegangenen Ansprüche, wobei das mindestens eine Halteelement (310) H-förmig ausgeformt ist.

11. Aufnahmevorrichtung (105) gemäß einem der vorangegangenen Ansprüche, die flexibel aus dem Reinigungsgerät (100) entnehmbar und/oder in das Reinigungsgerät (100) einsetzbar ist.

12. Reinigungsgerät (100), das die folgenden Merkmale aufweist:
- mindestens ein Korbelement (110) zum Aufnehmen von Reinigungsgut;
- eine Aufnahmevorrichtung (105) gemäß einem der vorangegangenen Ansprüche.

## Claims

1. Receiving means (105) for a cleaning device (100) for inserting into a basket element (110) of the cleaning device (100), the receiving means (105) being used to receive at least one tube (500) and comprising the following features:
- a plurality of retaining elements (310) each comprising a plurality of through-openings (320);
- a plurality of insert elements (330) which can each be inserted into one of the retaining elements (310) and each comprise a receiving region (400) which is designed to receive the tube (500);
- at least one connection element (700) which is designed to connect the tube (500) to a rinsing strip (200) of the cleaning device (100) in order to introduce a cleaning liquid into the tube (500),
**characterized in that**
the receiving means (105) comprises a frame unit (300) formed from a plurality of frame elements (305) and the plurality of retaining elements (310) assembled with the frame elements (305),
the insert elements (330) each comprising a first hooking element (440) in a first edge region (430) and a second hooking element (445) in a second edge region (435), which hooking elements are designed to hook the relevant insert element (330) into one of the retaining elements (310),
and a plurality of through-openings (320) of a relevant retaining element (310) each being arranged in a row on opposite legs (325) of the retaining element (310) for receiving the first hooking element (440) and the second hooking element (445).

2. Receiving means (105) according to claim 1, wherein the insert element (330) comprises a support lug (405) adjacent to the receiving region (400), in particular wherein the support lug (405) is formed integrally with the insert element (330), and/or wherein the support lug (405) is formed by a bent punched portion of the insert element (330).

3. Receiving means (105) according to either of the preceding claims, wherein the insert element (330) comprises at least one further receiving region (410) which is designed to hold a further tube (507).

4. Receiving means (105) according to claim 3, wherein the insert element (330) comprises a separating projection (420) between the receiving region (400) and the at least one further receiving region (410) for separating the receiving regions (400, 410).

5. Receiving means (105) according to claim 4, wherein the separating projection (420) is Y-shaped.

6. Receiving means (105) according to any of the preceding claims, comprising an extension element (1000) which can be inserted into the insert element (330), wherein the extension element (1000) has at least one extension receiving region (1010) which is designed to hold at least one additional tube.

7. Receiving means (105) according to claim 6, wherein the insert element (330) comprises at least one clipping element (450, 455) for fastening the extension element (1000) to the insert element (330).

8. Receiving means (105) according to any of the preceding claims, wherein the connection element (700) is connected, in particular screwed, to the rinsing strip (200).

9. Receiving means (105) according to any of the preceding claims, wherein the frame unit (300) is L-shaped in an assembled state.

10. Receiving means (105) according to any of the preceding claims, wherein the at least one retaining element (310) is H-shaped.

11. Receiving means (105) according to any of the preceding claims, which can be flexibly removed from the cleaning device (100) and/or can be inserted into the cleaning device (100).

12. Cleaning device (100) comprising the following features:
- at least one basket element (110) for receiving items to be cleaned;
- a receiving means (105) according to any of the preceding claims.

## Revendications

1. Dispositif de réception (105) pour un appareil de nettoyage (100), destiné à être inséré dans un élément formant panier (110) de l'appareil de nettoyage (100), dans lequel le dispositif de réception (105) sert à la réception d'au moins un tuyau flexible (500) et présente les caractéristiques suivantes :
- plusieurs éléments de maintien (310) qui présentent respectivement une pluralité d'ouvertures de passage (320) ;
- plusieurs éléments d'insertion (330) pouvant être insérés respectivement dans l'un des éléments de maintien (310), lesquels éléments d'insertion présentent respectivement une zone de réception (400) qui est formée afin de recevoir le tuyau flexible (500) ;
- au moins un élément de raccordement (700) qui est formé afin de raccorder le tuyau flexible (500) à un collecteur de lavage (200) de l'appareil de nettoyage (100) afin d'introduire un liquide de nettoyage dans le tuyau flexible (500),
**caractérisé en ce que**
le dispositif de réception (105) présente une unité formant ossature (300) formée de plusieurs éléments d'ossature (305) et des éléments de maintien (310) assemblés avec les éléments d'ossature (305),
dans lequel les éléments d'insertion (330) présentent respectivement, dans une première zone de bord (430), un premier élément d'accrochage (440) et, dans une seconde zone de bord (435), un second élément d'accrochage (445), lesquels sont conçus afin d'accrocher l'élément d'insertion (330) respectif dans l'un des éléments de maintien (310),
et dans lequel plusieurs ouvertures de passage (320) d'un élément de maintien (310) respectif sont respectivement disposées en une rangée sur des branches (325) opposées de l'élément de maintien (310) pour la réception du premier élément d'accrochage (440) et du second élément d'accrochage (445).

2. Dispositif de réception (105) selon la revendication 1, dans lequel l'élément d'insertion (330) présente un ergot de support (405) adjacent à la zone de réception (400), en particulier dans lequel l'ergot de support (405) est formé d'une seule pièce avec l'élément d'insertion (330), et/ou dans lequel l'ergot de support (405) est formé par une section poinçonnée repliée de l'élément d'insertion (330).

3. Dispositif de réception (105) selon l'une des revendications précédentes, dans lequel l'élément d'insertion (330) présente au moins une zone de réception supplémentaire (410) qui est conçue afin de maintenir un tuyau flexible supplémentaire (507).

4. Dispositif de réception (105) selon la revendication 3, dans lequel l'élément d'insertion (330) présente, entre la zone de réception (400) et l'au moins une zone de réception supplémentaire (410), un organe de séparation (420) pour la séparation des zones de réception (400, 410).

5. Dispositif de réception (105) selon la revendication 4, dans lequel l'organe de séparation (420) est formé en forme de Y.

6. Dispositif de réception (105) selon l'une des revendications précédentes, comportant un élément d'élargissement (1000) qui peut être inséré dans l'élément d'insertion (330), dans lequel l'élément d'élargissement (1000) présente au moins une zone de réception d'extension (1010) qui est conçue afin de maintenir au moins un tuyau flexible additionnel.

7. Dispositif de réception (105) selon la revendication 6, dans lequel l'élément d'insertion (330) présente au moins un élément de clipsage (450, 455) pour la fixation de l'élément d'extension (1000) à l'élément d'insertion (330).

8. Dispositif de réception (105) selon l'une des revendications précédentes, dans lequel l'élément de raccordement (700) est relié, en particulier vissé, au collecteur de lavage (200).

9. Dispositif de réception (105) selon l'une des revendications précédentes, dans lequel l'unité formant ossature (300) est formée en forme de L dans un état assemblé.

10. Dispositif de réception (105) selon l'une des revendications précédentes, dans lequel l'au moins un élément de maintien (310) est formé en forme de H.

11. Dispositif de réception (105) selon l'une des revendications précédentes, lequel peut être retiré de l'appareil de nettoyage (100) et/ou inséré dans l'appareil de nettoyage (100) de manière flexible.

12. Appareil de nettoyage (100) qui présente les caractéristiques suivantes :
- au moins un élément formant panier (110) pour la réception d'articles à nettoyer ;
- un dispositif de réception (105) selon l'une des revendications précédentes.
